# EUROPÄISCHE PATENTSCHRIFT

(11) **EP 0 942 758 B1**
(45) Veröffentlichungstag und Bekanntmachung des Hinweises auf die Patenterteilung: **14.01.2004**
(21) Anmeldenummer: 97951919.6
(22) Anmeldetag: 19.11.1997
(51) Int. Cl.: A61L 27/00, A61P 19/00

(54) **VERBINDUNGEN MIT VERBESSERTER KNORPEL- UND/ODER KNOCHENINDUZIERENDER AKTIVITÄT**
COMPOUNDS WITH IMPROVED INDUCTIVE EFFECT ON CARTILAGE AND BONES
COMPOSES A ACTION INDUCTRICE AMELIOREE SUR LES CARTILAGES ET LES OS

(30) Priorität: 19.11.1996 DE 19647853
(43) Veröffentlichungstag der Anmeldung: 22.09.1999
(73) Patentinhaber: BIOPHARM GESELLSCHAFT ZUR BIOTECHNOLOGISCHEN ENTWICKLUNG VON PHARMAKA mbH, 69115 Heidelberg (DE)
(72) Erfinder: PAULISTA, Michael, D-69181 Leimen (DE); POHL, Jens, D-76707 Hambrücken (DE); PABST, Joachim, D-64354 Reinheim (DE); HEIDE, Helmut, D-65779 Kelkheim (DE)
(74) Vertreter: Böhm, Brigitte, Dipl.-Chem. Dr.
(86) Internationale Anmeldenummer: PCT/EP1997/006463
(87) Internationale Veröffentlichungsnummer: WO 1998/021972

(56) Entgegenhaltungen:
- WO-A-95/04819
- DE-A- 3 810 803
- US-A- 4 596 574
- US-A- 4 681 763

### SEQUENZPROTOKOLL

(1) ALLGEMEINE ANGABEN:
   (i) ANMELDER:
      (A) NAME: Biopharm Gesellschaft zur biotechnologischen Entwicklung von Pharmaka mbH
      (B) STRASSE: Czernyring 22
      (C) ORT: Heidelberg
      (E) LAND: Deutschland
      (F) POSTLEITZAHL: 69115

      (A) NAME: GerontoCare GmbH Biomaterials & Medical Devices
      (B) STRASSE: Rossbergring 107
      (C) ORT: Reinheim/odw.
      (E) LAND: Deutschland
      (F) POSTLEITZAHL: 64354
   (ii) BEZEICHNUNG DER ERFINDUNG:
      Verbindungen mit verbesserter knorpel- und/oder knocheninduzierender Aktivitaet
   (iii) ANZAHL DER SEQUENZEN: 1
   (iv) COMPUTER-LESBARE FASSUNG:
      (A) DATENTRÄGER: Floppy disk
      (B) COMPUTER: IBM PC compatible
      (C) BETRIEBSSYSTEM: PC-DOS/MS-DOS
      (D) SOFTWARE: Patent In Release #1.0, Version #1.30 (EPA)
   (vi) DATEN DER URANMELDUNG:
      (A) ANMELDENUMMER: DE 19647853.7
      (B) ANMELDETAG: 19-NOV-1996
(2) ANGABEN ZU SEQ ID NO: 1 :
   (i) SEQUENZKENNZEICHEN:
      (A) LÄNGE: 501 Aminosäuren
      (B) ART: Aminosäure
      (C) STRANGFORM:
      (D) TOPOLOGIE: linear
   (ii) ART DES MOLEKÜLS: Protein
   (xi) SEQUENZBESCHREIBUNG: SEQ ID NO: 1:
   Met Arg Leu Pro Lys Leu Leu Thr Phe Leu Leu Trp Tyr Leu Ala Trp
   1               5                   10                  15
   Leu Asp Leu Glu Phe Ile Cys Thr Val Leu Gly Ala Pro Asp Leu Gly
               20                  25                  30
   Gln Arg Pro Gln Gly Thr Arg Pro Gly Leu Ala Lys Ala Glu Ala Lys
           35                  40                  45
   Glu Arg Pro Pro Leu Ala Arg Asn Val Phe Arg Pro Gly Gly His Ser
       50                  55                  60
   Tyr Gly Gly Gly Ala Thr Asn Ala Asn Ala Arg Ala Lys Gly Gly Thr
   65                  70                  75                  80
   Gly Gln Thr Gly Gly Leu Thr Gln Pro Lys Lys Asp Glu Pro Lys Lys
                   85                  90                  95
   Leu Pro Pro Arg Pro Gly Gly Pro Glu Pro Lys Pro Gly His Pro Pro
               100                 105                 110
   Gln Thr Arg Gln Ala Thr Ala Arg Thr Val Thr Pro Lys Gly Gln Leu
           115                 120                 125
   Pro Gly Gly Lys Ala Pro Pro Lys Ala Gly Ser Val Pro Ser Ser Phe
       130                 135                 140
   Leu Leu Lys Lys Ala Arg Glu Pro Gly Pro Pro Arg Glu Pro Lys Glu
   145                 150                 155                 160
   Pro Phe Arg Pro Pro Pro Ile Thr Pro His Glu Tyr Met Leu Ser Leu
                   165                 170                 175
   Tyr Arg Thr Leu Ser Asp Ala Asp Arg Lys Gly Gly Asn Ser Ser Val
               180                 185                 190
   Lys Leu Glu Ala Gly Leu Ala Asn Thr Ile Thr Ser Phe Ile Asp Lys
   195 200 205
   Gly Gln Asp Asp Arg Gly Pro Val Val Arg Lys Gln Arg Tyr Val Phe
   210 215 220
   Asp Ile Ser Ala Leu Glu Lys Asp Gly Leu Leu Gly Ala Glu Leu Arg
   225 230 235 240
   Ile Leu Arg Lys Lys Pro Ser Asp Thr Ala Lys Pro Ala Ala Pro Gly
   245 250 255
   Gly Gly Arg Ala Ala Gln Leu Lys Leu Ser Ser Cys Pro Ser Gly Arg
   260 265 270
   Gln Pro Ala Ser Leu Leu Asp Val Arg Ser Val Pro Gly Leu Asp Gly
   275 280 285
   Ser Gly Trp Glu Val Phe Asp Ile Trp Lys Leu Phe Arg Asn Phe Lys
   290 295 300
   Asn Ser Ala Gln Leu Cys Leu Glu Leu Glu Ala Trp Glu Arg Gly Arg
   305 310 315 320
   Ala Val Asp Leu Arg Gly Leu Gly Phe Asp Arg Ala Ala Arg Gln Val
   325 330 335
   His Glu Lys Ala Leu Phe Leu Val Phe Gly Arg Thr Lys Lys Arg Asp
   340 345 350
   Leu Phe Phe Asn Glu Ile Lys Ala Arg Ser Gly Gln Asp Asp Lys Thr
   355 360 365
   Val Tyr Glu Tyr Leu Phe Ser Gln Arg Arg Lys Arg Arg Ala Pro Leu
   370 375 380
   Ala Thr Arg Gln Gly Lys Arg Pro Ser Lys Asn Leu Lys Ala Arg Cys
   385 390 395 400
   Ser Arg Lys Ala Leu His Val Asn Phe Lys Asp Met Gly Trp Asp Asp
   405 410 415
   Trp Ile Ile Ala Pro Leu Glu Tyr Glu Ala Phe His Cys Glu Gly Leu
   420 425 430
   Cys Glu Phe Pro Leu Arg Ser His Leu Glu Pro Thr Asn His Ala Val
   435 440 445
   Ile Gln Thr Leu Met Asn Ser Met Asp Pro Glu Ser Thr Pro Pro Thr
   450 455 460
   Cys Cys Val Pro Thr Arg Leu Ser Pro Ile Ser Ile Leu Phe Ile Asp
   465 470 475 480
   Ser Ala Asn Asn Val Val Tyr Lys Gln Tyr Glu Asp Met Val Val Glu
   485 490 495
   Ser Cys Gly Cys Arg
   500

## Beschreibung

Die vorliegende Erfindung betrifft neue verbesserte Verbindungen mit knorpelund/oder knocheninduzierender Aktivität, bestehend aus einem oder mehreren Mitgliedern der TGF-β Familie, vorzugsweise MP52, oder einer hierfür kodierenden DNA-Sequenz und einer speziellen Trägermatrix aus kristallographisch phasenreinem Tricalciumphosphat. Die Erfindung betrifft weiterhin die Herstellung dieser Verbindungen und Verwendung zur Behandlung von Erkrankungen die den Knorpel und/oder Knochen betreffen sowie zur Behandlung von Schädigungen des Knorpel- und/oder Knochengewebes.

Viele Wachstumsfaktoren aus der TGF-β-Superfamilie sind für einen weiten Bereich medizinischer Behandlungsmethoden und Anwendungen, die insbesondere Wundheilung und Gewebewiederherstellung betreffen, relevant. Für einen Überblick über Mitglieder der TGF-β-Superfamilie vgl. z.B.: Roberts, A.B. & Sporn, M.B. Handbook of Experimental Pharmacology 95 (1990) 419-472; Kingsley , D.M., Genes & Development 8 (1994) 133-146 und die darin zitierte Literatur. Zu den Mitgliedern zählen die TGF-β Proteine, wie das TGF-β1, TGF-β2, TGF-β3, TGF-β4 und TGF-β5, vgl. z.B.: U.S. Patent 5,284,763; EP 0376785; U.S. Patent 4,886,747; Madisen, L. et al., DNA 7 (1988) 1-8; Derynck, R. et al., EMBO J. 7 (1988) 3737-3743; Jakowlew, S.B. et al., Mol. Endo. 2 (1988) 1186-1195; Kondaiah, P. et al., J. Biol. Chem. 265 (1990) 1089-1093. Eine weitere Unterfamilie bilden die Aktivine/Inhibine mit den bislang bekannten Aktivinketten βA, βB, βC und βD, vgl. z:B.: Mason, A.J. et al., Biochem. Biophys. Res. Commun. 135 (1986) 957-964; Hötten, G. et al., Biochem. Biophys. Res. Commun. 206 (1995) 608-613; Oda, S. et al., Biochem. Biophys. Res. Comm. 210 (1995) 581-588. Auch GDF-12 kann aufgrund seiner Aminosäurehomologie dieser Unterfamilie zugeordnet werden (vgl. WO 96/02559). Von ßA und ßB ist bekannt, daß sie neben dem Homodimer auch ein Heterodimer βAβB bilden können. Bei Kombination mit einer α Untereinheit entstehen die Inhibine, die im wesentlichen entgegengesetzte Aktivitäten in Vergleich zu Aktivinen aufweisen, vgl.: Vale, W. et al., Handbook of Experimental Pharmacology 95 (1990) 211-248; Vale, W. et al., The Physiology of Reproduction, Raven Press, New York (1994) 1861-1878. Eine weitere Unterfamilie bilden die Mitglieder der BMP (Bone Morphogenetic Protein)- Familie wozu die Proteine BMP-2 (BMP-2a), BMP-3, BMP-3b, BMP-4 (BMP-2b), BMP5, BMP-6, BMP-7 (OP-1), BMP-8 (OP-2), BMP-9, BMP-10, BMP-11, BMP-12 und BMP-13 zählen, vgl. z.B.: Wozney, J.M. et al. Science 242 (1988) 1528-1534; Celeste, A.J. et al., Proc. Natl. Acad. Sci. USA 87 (1990) 9843-9847; Özkaynak, E. et al., J. Biol. Chem. 267 (1992) 25220-25227; Takao et al. Biochem. Biophys. Res. Com. 219 (1 996) 656-662; WO 93/00432; WO 94/26893; WO 94/26892, WO 95/16035. Eine weiter Untergruppe ist die GDF (Growth Differentiation Factor)-Familie, zu denen GDF-1, GDF-3, GDF-9, GDF-10, GDF-1 1 sowie die für die Knorpel- und/oder Knocheninduktion insbesondere interessanten GDF-5, GDF-6 und GDF-7 zählen; vgl.: McPherron, A.C. & Lee, S.-J., J. Biol. Chem. 268 (1993) 3444-3449; Storm, E.E. et al., Nature 368 (1994) 639-643; Lee, S.-J.; Proc. Natl. Acad. Sci. USA 88 (1991) 4250-4254; Cunningham et al. Growth Factors 12 (1995), 99-109; Hötten, G. et al., Growth Factors 13 (1996) 65-74; Chang, S.C. et al., J. Biol. Chem. 269 (1994) 28227-28234. Zwischen den Untergruppen der GDF und BMP Familie gibt es aufgrund von Aminosäurehomologien teilweise Überlappungen. Für die TGF-β Superfamilienmitglieder dpp und 60A aus Drosophila konnte auch ein knorpel- und knocheninduzierendes Potential nachgewiesen werden, vgl.: Sampath, T.K. et al., Proc. Natl. Acad. Sci. USA 90 (1993) 6004-6008. Interessant sind auch die Proteine dorsalin und das Bone Formation-Inducing Protein, vgl.: Basler, K. et al., Cell 73 (1993) 687-702; WO 94/01557. Beschrieben sind auch Heterodimere von verschiedenen Mitgliedern, vgl. z.B:: Aono, A. et al., Biochem. Biophys. Res. Commun. 210 (1995) 670-677; WO 93/09229; EP 0 626 451. Bekannt ist, daß viele Mitglieder insbesondere aus den Unterfamilien der TGF-β-, BMP- und GDF-Familien ein knorpelund/oder knocheninduzierendes Potential aufweisen, wobei aber auch Mitglieder der Aktivinfamilie, zumindest in Kombination mit weiteren TGF-β-Superfamilienmitgliedern, Einfluß auf die Knochenbildung nehmen können; vgl. beispielsweise Hock, J.M. et al., Endocrinol. 126 (1990) 421-426; Wang et al., Proc. Natl. Acad. Sci. USA 87 (1990) 2220-2224; Wozney et al., Mol. Reprod. Dev. 32 (1992) 160-167; Sampath et al., J. Biol. Chem. 267 (1992) 20352-20362; Ogawa, Y. et al., J. Biol. Chem. 267 (1992) 14233-14237; WO 88/00205; US-PS 5,013,649; WO 89/10409: WO 90/11366; WO 91/05802; WO 92/15323; WO 91/18098; WO 93/00432; WO 93/09229; WO 94/01557; WO 94/26893; WO 94/26892; WO 94/15949; WO 95/01801; WO 95/01802 und EP 0 626 451. Da die einzelnen Proteine teilweise an unterschiedlichen Stellen im Verlauf der Knorpel- und Knocheninduktion wirken, ist davon auszugehen, daß die Kombination verschiedener dieser Proteine vorteilhaft für die Effizienz der Knorpel- und Knocheninduktion ist. Derartige Proteingemische sind innerhalb dieser Erfindung mitumfaßt.

In WO 93/16099, WO 95/04819 und WO 96/01316 werden die DNA- und Proteinsequenzen von Proteinen der TGF-β-Familie, nämlich dem MP52 und MP121, beschrieben. Bei MP121 handelt es sich um das bereits oben genannte Aktivin βC. Insbesondere von Interesse ist MP52 (in Publikationen teilweise auch GDF5 genannt), für das u.a. bereits ein knorpel- und knocheninduzierendes Potential nachgewiesen werden konnte (WO 95/04819 und Hötten et al. Growth Factors 13 (1996) 65-74).

Die Mitglieder der TGF-β Superfamilie, die ein knorpel- und/oder knocheninduzierendes Potential besitzen, zeichnen sich im reifen Anteil durch hohe Aminosäurehomologien aus und besitzen die für TGF-β Superfamilienmitglieder typischen sieben konservierten Cysteine. Mitglieder dieser Superfamilie liegen in ihrer aktiven Form normalerweise alle als homo- und/oder heterodimere Proteine vor. Das knorpel- und/oder knocheninduzierende Potential dieser Proteine wird meistens auf inerten Trägermatrices, die selber keinerlei knorpel- und/oder knocheninduzierende Wirkung aufweisen, nachgewiesen.

Bereits in den 60er Jahren begannen intensive Forschungsarbeiten über die Einsatzmöglichkeiten von Calciumphosphat-Keramiken für den implantierbaren Knochenersatz (Bhaskar et al., Oral Surg. 32 (1971) 47), denen die chemische Analogie dieser Verbindungsgruppe zu dem mineralischen Anteil des Knochens zu Grunde lag. Eine der ersten systematischen Untersuchungen über die Zusammenhänge zwischen den chemisch-werkstofflichen Parametern und den biologischen Eigenschaften wurde Anfang der 70er Jahre am Battelle Institut durchgeführt (Heide, Köster et al., Z. Orthop. 118 (1979) 398 und Biotechn. Umschau 2 (1978) 226). Hierbei wurden Calciumphosphate mit wechselnden CaO/P₂O₅-Verhältnissen durch Sinterverfahren als granuläre und stückige keramische Implantatmaterialien hergestellt und im Tierversuch getestet. Die herausragenden Ergebnisse dieser Studien lassen sich wie folgt zusammenfassen:
(a) Calciumphosphatkeramiken innerhalb bestimmter Zusammensetzungen zeichnen sich durch eine hervorragende Gewebeverträglichkeit gegenüber Knochen aus.
(b) Das Optimum der Gewebeverträglichkeit konzentriert sich auf Keramiken mit einem CaO/P₂O₅-Verhältnis wie 3/1, also auf das Tricalciumphosphat TCP, Ca₃(PO₄)₂ (bzw. in keramischer Formelschreibweise 3CaO·P₂O₅) und den Hydroxylapatit (HA) selbst, also [Ca₅(PO₄)₃OH], der sich ebenfalls synthetisch darstellen lässt. Dieses Ergebnis ist folgerichtig, da bekanntlich auch die Zusammensetzung des mineralischen Knochenanteiles mit seiner wichtigsten mineralischen Komponente, dem Hydroxylapatit diesem Verhältnis in etwa entspricht. Obwohl TCP und HA eine ähnlich chemische Zusammensetzung besitzen, unterscheiden sie sich in ihrem Löslichkeitsverhalten und anderen physikalischen Eigenschaften, wie der Dichte und der Festigkeit erheblich voneinander. Hiervon hängen naturgemäß auch die möglichen Einsatzgebiete ab.
(c) Die beiden optimal biokompatiblen Modifikationen des Tricalciumphosphats TCP (d.h. die metastabile Hochtemperaturmodifikation α-TCP und besonders die stabile Tieftemperaturmodifikation β-TCP) und der Hydroxylapatit HA sind mehr oder weniger biodegradabel, d.h. sie werden im biologischen Lager mehr oder weniger schnell abgebaut bzw. resorbiert. Eine ausgeprägte Biodegradabilität weisen nach Ramselaar et al., J. Materials Sci. 2 (1991) 63, α- und β-TCP auf. HA unterliegt im biologischen Milieu einer sehr viel geringeren Resorption. Der Abbau des TCP im Knochenlager erfolgt nach Untersuchungen mit radioaktiv dotierten Implantatmaterialien von Schuster, Heide et. al., (unveröffentl. Ber. des Battelle Institutes Frankfurt) auf mehr chemischem Wege, d.h. Lösung und Verstoffwechselung der Lösungsprodukte erfolgt ohne Beteiligung knochenabbauender Zellen, während die sehr viel langsamere Resorption des Hydroxylapatites mehr auf der spezifischen Leistung knochenabbauender Zellen (Osteoklasten) beruht.
(d) Die biokompatiblen Calciumphosphatkeramiken auf der Basis von TCP und HA werden im Knochenlager weitgehend ohne bindegewebige Abkapselung integriert, wie in den 70er Jahren u.a. von der genannten Battelle-Arbeitsgruppe im Tierexperiment eindrucksvoll belegt werden konnte. Für diese herausragende Eigenschaft hat man damals den Ausdruck "Bioaktivität" eingeführt.

Im Zuge der weiteren Entwicklung der vielversprechenden Calciumphosphatkeramiken erwies sich eine detaillierte Kenntnis der komplexen kristallchemischen Zusammenhänge des Systems CaO-P₂O₅ ( + H₂O) als unumgängliche Voraussetzung für eine systematische Optimierung. Leider ist in der Vergangenheit und wird noch immer von zahlreichen Anwendern gegen diese Voraussetzung verstoßen, insbesondere dann, wenn für typische temporäre Anwendungen wie z.B. die Sanierung von Parodontaltaschen Materialien auf der Basis des nur schwer biodegradablen HA eingesetzt werden. Wichtige Arbeiten in diesem Zusammenhang wurden von De Groot et al., Biomaterials 1 (1980) 47, und Bauer und Hohenberger, Berichte der DKG 66 (1989) 23, veröffentlicht.

Zahlreiche, heute noch marktübliche Implantatmaterialien, welche aus undefinierten Gemengen von TCP und HA sowie anderen Calciumphosphat-Phasen wie Dioder Tetracalciumphosphaten und Calciumphosphatgläsern bestehen, haben negative biomedizinische Eigenschaften im Sinne einer Provokation von Bindegewebsinfiltrationen sowie Aktivierung von Makrophagen, mitunter begleitet von entzündlichen Reaktionen. Die bindegewebige Einkapselung solcher fehlerhaft zusammengesetzter Materialien ist dann Ausdruck der Abstoßung des Implantats (Bauer und Hohenberger, Berichte der DKG 66 (1989) 23). Die stöchiometrische Zusammensetzung allein ist kein Kriterium für die Existenz von unphysiologischen Fremdphasen. Aus diesen Erkenntnissen leitet sich die Forderung nach kristallographischer Phasenreinheit der jeweils verwendeten Implantatmaterialien ab.

Die beiden Haupttypen des Calciumphosphats, das Tricalciumphosphat (TCP) und der Hydroxylapatit (HA) haben entsprechend ihrer unterschiedlichen Resorption unterschiedliche Einsatzgebiete: TCP ist insbesondere vorteilhaft für den temporären Knochenersatz, bei dem im Laufe der Zeit eine Resorption des Biomateriales simultan mit der Knochenregeneration erfolgt (Zystenfüllung im Kieferbereich, Auffüllen krankheits- bzw. operationsbedingter oder degenerativer Knochendefekte usw.). HA dagegen ist vorzugsweise bei langdauerndem Knochenersatz angezeigt, wie z.B. in Verbindung mit der Beschichtung von Gelenkendoprothesen, bei denen man einen direkten Kontakt des belasteten Knochenlagers mit dem Metall oder anderen inerten Materialien vermeiden will.

Der vorliegenden Erfindung liegt die Aufgabe zugrunde, neue Verbindungen bereitzustellen, die besonders hohe knorpel- und/oder knocheninduzierende Aktivitäten in Säugetieren und insbesondere Primaten wie dem Menschen aufweisen, aber nicht oder in möglichst geringem Umfang die Nachteile der bisher verwendeten Materialien aufweisen. Solche Verbindungen sollen ermöglichen, den Heilungsprozess von Erkrankungen, die den Knorpel und/oder Knochen betreffen und die insbesondere mit einem Verlust an Knochensubstanz einhergehen, und/oder von Schädigungen des Knorpel- und/oder Knochengewebes wesentlich zu beschleunigen.

Diese Aufgabe wird erfindungsgemäß gelöst durch ein bioaktives Implantatmaterial für den Knochenersatz mit Knorpel- und/oder Knochen-bildender Aktivität bestehend aus zwei Komponenten A und B, welches als Komponente A ein auf B aufgebrachtes knorpel- und/oder knocheninduzierendes Protein oder Proteingemisch oder für ein solches Protein oder Proteingemisch kodierende DNA, und als Komponete B ein Matrixmaterial aus Calciumphosphat aufweist, welches in einer für die medizinische Anwendung hergestellten injizierbaren Suspension in geeigneten Flüssigkeiten selbst osteoinduktiv ist. Bevorzugte Ausgestaltungen der Erfindung sind in den Unteransprüchen beschrieben. Insbesondere wird ein Material bereitgestellt, das aus den zwei Komponenten A und B besteht, wobei A ein Protein oder Proteingemisch, und zwar aus einem oder mehreren homo- oder heterodimeren Proteinen der TGF-β-Superfamilie mit knorpel- und/oder knocheninduzierender Aktivität bedeutet, und B ein Matrixmaterial aus Calciumphosphat, besonders α- oder β-Tricalciumphosphat-Keramik, bedeutet, welches in einer für die medizinische Anwendung hergestellten, injizierbaren Suspension in geeigneten Flüssigkeiten selbst osteoinduktiv ist. A ist dabei an B assoziiert, ohne kovalent gebunden zu sein und kann z.B. während des Knochenbildungsprozesses langsam von B in dem Maße freigesetzt werden, wie B dem chemischen Abbau im Knochenlager unterliegt. Damit wird A einem sogenannten "controlled release" unterworfen.

Alternativ kann A auch eine für die genannten Proteine oder Proteingemische kodierende DNA bedeuten. Die DNA kann gegebenenfalls vor Degeneration nach dem Fachmann bekannten Methoden geschützt werden. Eine derartige DNA kann nach Freisetzung in das umliegende Gewebe von den dort vorhandenen Zellen oder aber von in die Trägermatrix einwandernden Zellen aufgenommen und exprimiert werden, sodass als wirksamer Stoff wiederum die exprimierten Proteine oder Proteingemische agieren.

Vorzugsweise ist die DNA deshalb mit Sequenzen assoziiert, die eine Expression bewirken oder fördern. Die Förderung der Expression ist insbesondere durch gezielte Rekombination in das Zellgenom möglich und zwar an eine Stelle, die zu einer Erzeugung von Protein unter Kontrolle zellulärer Sequenzen führt.

Andererseits ist auch der Einsatz von DNA auf einem geeigneten Expressionsvektor möglich.

Der Begriff "Protein der TGF-β-Superfamilie mit knorpel- und/oder knocheninduzierender Aktivität" bedeutet ein Protein, das im reifen Anteil die charakteristischen konservierten 7 Cysteine enthält. Dazu zählen Mitglieder der TGF-β-, der Aktivin-, der BMP- und der GDF- Familie und insbesondere MP52 sowie Fragmente davon mit grundsätzlich derselben Aktivität. Die entsprechenden Nukleotid- und Proteinsequenzen sind aus den oben genannten Referenzen, auf deren Offenbarung hiermit Bezug genommen wird, zu entnehmen. Umfaßt sind bevorzugt Homodimere der genannten Proteine aber auch Heterodimere aus verschiedenen Familienmitgliedern. Bevorzugt umfaßt sind Proteine, die denselben Rezeptormechanismus und/oder dieselbe Signalübertragung wie die Mitglieder der BMP- und/oder GDF-Familie, insbesondere von MP52, besitzen. Umfaßt ist auch die Kombination von verschiedenen Proteinen der TGF-β-Superfamilie mit knorpel- und/oder knocheninduzierender Aktivität. Das knorpel- und/oder knocheninduzierende Potential kann in bekannten Versuchen wie z.B. *in vivo* durch Induktion von Knorpel und/oder Knochen nach Implantation des Proteins mit einer geeigneten Trägermatrix in die Rattenmuskulatur; vgl. z.B. Sampath, T.K. et al., J. Biol. Chem. 267 (1992) 20352-20362 und/oder *in vitro* durch Induktion von Alkalischer Phosphatase Aktivität auf ROB-C26 Zellen; vgl. Yamaguchi, A. et al., J. Cell Biol. 113 (1991) 681-687 und/oder W-20-17 Zellen; vgl.: Thies, R.S, et al. Endocrinol. 130 (1992) 1318-1324 und/oder Stimulation der Expression von Proteinen der extrazellulären Matrix, vgl.: Hock, J.M. et al. Endocrinol. 126 (1990) 421-426 und/oder in Versuchen wie sie bei Chen, P. et al., Exp. Cell Res. 195 (1991) 509-515 und/oder Vukicevic, S., et al. Proc. Natl. Acad. Sci. USA 86 (1989) 8793-8797 beschrieben sind, überprüft werden. Das Protein kann als reifes Protein aber auch als Vorläufer-Protein oder Protein mit verschiedenen Prozessierungen im Propeptidanteil und/oder mit zusätzlichen oder veränderten N- und/oder C-terminalen Aminosäuresequenzen, welche die biologische Aktivität im wesentlichen nicht beeinflussen, vorliegen.

Andererseits sind auch Fusionsproteine möglich, wo neben dem für das reife Protein kodierenden Anteil oder Fragmente(n) davon auch noch funktionelle Signal- oder/und Propeptidanteile von anderen Proteinen, insbesondere der TGF-β Superfamilie, insbesondere auch der Aktivin-, BMP- und GDF-Proteine umfaßt sind. Die entsprechenden Nukleotid- und Proteinsequenzen sind aus den oben genannten Referenzen zu entnehmen, auf deren Offenbarung hiermit Bezug genommen wird. Wichtig ist, daß der richtige Leserahmen für das reife Protein erhalten bleibt. So ist z.B. der Austausch von Propeptidanteilen durch entsprechende Anteile anderer Proteine bei Mol. Endocrinol. 5 (1991), 149-155 und Proc. Natl. Acad. Sci. USA 90 (1993), 2905-2909 beschrieben.

Das Protein in der erfindungsgemäßen Verbindung oder das hiervon kodierte Protein kann substituierte oder eingefügte Aminosäuren enthalten oder deletiert sein, ebenfalls unter der Voraussetzung, daß die Aktivität nicht wesentlich beeinflußt wird, und aus verschiedenen Spezies, wie z.B. Mensch, Maus, Ratte, Rind oder Schwein isoliert sein. Ferner kann das Protein durch im Stand der Technik bekannte Methoden, beispielsweise Glycosylierungen, Phosphatierungen, Sulfatierungen und Veresterung mit Fetten modifiziert sein, ebenfalls unter der Bedingung, daß sich daraus keine wesentliche Veränderung der Aktivität ergibt.

In einer bevorzugten Ausführungsform der vorliegenden Erfindung ist A ein Protein aus der GDF- oder BMP-Familie oder ein Fragment davon.

In einer besonders bevorzugten Ausführungsform der vorliegenden Erfindung ist die Komponente A charakterisiert durch ein Protein, das
(a) den reifen Anteil und gegebenenfalls weitere funktionelle Anteile der in SEQ ID NO. 1 gezeigten Proteinsequenz enthält,
(b) Teile des reifen Anteils von (a) enthält, die im Wesentlichen dieselbe Aktivität aufweisen, insbesondere reife Proteine mit verändertem N-Terminus,
(c) Teile entsprechend (a) oder (b) enthält, die aufgrund der Herkunft des Proteins aus anderen Wirbeltieren von SEQ ID NO.1 abweichen, aber im Wesentlichen dieselbe Aktivität aufweisen,
(d) neben Teilen des reifen Proteins gemäß (a), (b) oder (c) noch Teile eines anderen Proteins aus der TGF-β-Superfamilie in Form eines Fusionsproteins enthält,
(e) neben monomeren reifen Proteinen gemäß (a) bis (d) noch ein Monomer eines anderen Proteins aus der TGF-β-Superfamilie unter Ausbildung von Heterodimeren enthält,
(f) neben dimeren reifen Proteinen gemäß (a) bis (e) noch mindestens ein Dimer eines anderen Proteins aus der TGF-β-Superfamilie enthält.

Insbesondere umfasst diese Ausführungsform das reife Protein MP52 oder funktionelle Anteile bzw. Fragmente davon, wobei die aktive Form vorzugsweise als Dimer vorliegt. Besonders bevorzugt sind funktionelle Teilbereiche bzw. Abschnitte bzw. Fragmente, die mindestens den Bereich der sieben konservierten Cysteine enthalten.

Eine "biokompatible" und "bioaktive" Trägermatrix bedeutet im osteologischen Sinne eine Calciumphosphat-Keramik, welche einerseits ohne schädliche Gewebereaktionen wie bindegewebige Abkapselungen, Entzündungen und Gewebeentartungen in den Knochen integriert werden kann und andererseits ein direktes Aufwachsen von Knochen auf bzw. in die Oberflächenstruktur des Implantats stimuliert. Die eigentliche "Bioaktivität" einer Trägermatrix liegt jedoch erst dann vor, wenn histologisch wie klinisch nachweisbar eine Stimulation des Knochenwachstums zustande kommt. Für die erfindungsgemäße hochporöse bioaktive Trägermatrix (wie z.B. Cerasorb® ) auf Basis von Tricalciumphosphat, insbesondere von β- und auch α-TCP, liegen klinische Erfahrungen vor. Eine herausragende Stellung bezüglich Bioaktivität oder Osteoinduktivität nimmt das phasenreine und offen mikroporöse ß-TCP ein, welches allein durch die vorhersagbare chemische Auflösung im Knochenlager ein lonenmilieu erzeugt, das zur Stimulation der Osteoblastentätigkeit beiträgt und in situ als Substrat für die Osteoblastentätigkeit dient. Verläuft die chemische Auflösung oder Resoprtion der Trägermatrix simultan mit der Primärphase des Knochenaufbaus ("woven bone phase"), besteht die hervorragende Möglichkeit der Wiedergewinnung der Festigkeit und Struktur des umgebenden Knochenlagers. Voraussetzung hierfür ist die Abwesenheit unstöchiometrischer oft unphysiologisch reagierender Nebenphasen. Für phasenreines β-TCP kann dies nachgewiesen werden, da es als injizierbare Suspension in für die medizinische Anwendung geeigneten Flüssigkeiten, wie beispielsweise Serum, Plasma und Blut, allein schon osteoinduktiv wirksam ist, d.h. dass es zur Stimulation der Osteoblastentätigkeit beiträgt. Besonders bevorzugt sind kristallographisch phasenreine α- oder β-Tricalciumphosphat-Keramiken mit einer interkonnektierenden Mikroporosität im Bereich von 20 - 60 % ihres Volumens. In einer besonders bevorzugten Ausführungsform liegt die Primärkorngröße der kristallographischen phasenreinen α- oder β-Tricalciumphosphat-Keramik im Bereich von 10 - 40 µm. In einer weiteren bevorzugten Ausführungsform der Erfindung liegt dieses Implantatmaterial in Form einer injizierbaren Suspension vor. Es wird dadurch beispielsweise ermöglicht, das Material minimal-invasiv zu applizieren. Dabei verursacht die Suspension dieser Matrix in für die medizinische Anwendung geeigneten Flüssigkeiten, wie Wasser, Serum, Plasma und Blut, keine Riesenzell- oder Bindegewebeinfiltration in das Implantat.

Der wesentliche Gegenstand der Erfindung ist also ein Implantatmaterial aus zwei Komponenten A und B, in welchem die osteopoetische Wirkung der Komponente A durch eine osteoinduktive Wirkung der Komponente B synergistisch verstärkt wird. Der Verbindung liegt die vorteilhafte Kombination von Wirkungsmechanismen zweier Komponenten, d.h. knorpel- und/oder knocheninduzierendes Protein oder Proteingemisch und osteoinduktiver Trägermatrix, zugrunde. Ein solches erfindungsgemäßes Implantatmaterial vermeidet kontraproduktive Effekte gegenüber der osteopoetischen Wirkung der Proteine A, den manche biokompatible, aber nicht bioaktive Implantatmateriafien zeigen. So eignen sich Trägermaterialien wie HA aufgrund ihrer langsamen Biodegradabilität oft nicht zum Einsatz einer Protein-stimulierten Osteosynthese. Rasch biodegradable Trägerkeramiken wie Phosphatgläser und metastabile Phasen bzw. Phasengemische der CaP aber auch chemisch umgewandelte Matrizen, welche z.B. aus Korallen gewonnen werden, wirken allein schon durch die Aktivierung von Makrophagen oder/und Osteoklasten kontraproduktiv auf die Protein-stimulierte Osteosynthese. Ferner zeigte sich, daß die Belegung der Matrixoberfläche geeigneter Trägermaterialien mit physiologisch inerten Proteinfüllstoffen wie Kollagen, sich Resorptionsund damit Bioaktivitäts-hemmend auswirkte. Überraschenderweise erwiesen sich Mischungen von Trägermaterialien auf der Basis von mikroporösem phasenreinem TCP, vorzugsweise β-TCP, mit Homogenaten von rotem Knochenmark oder Blut, trotz der erheblichen Belegung der Matrixoberfläche mit Proteinen, als Osteosynthese-fördernd. Somit stellen die erfindungsgemäßen Implantationsmaterialien eine Optimierung dieser Befunde dar. Die minimale Matrixbelegung mit der Protein- oder DNA-Komponente A gewährleistet den Erhalt der bioaktiven, also eigenständigen osteoinduktiven Eigenschaften der Trägermatrix B und wird von dieser durch ihre interkonnektierende Mikroporenstruktur zwangsläufig in dem Maße freigesetzt und damit biologisch wirksam, wie es dem chemischen Abbau der Matrix am lmplantationsort entspricht. Das erfindungsgemäße osteopoetisch wirkende Protein A allein würde ohne die Kombination mit einer geeigneten Matrix durch Verstoffwechselung, Abtransport durch Körperflüssigkeiten oder ggf. Phagozytose rasch die biologische Wirkung am Implantationsort verlieren. Die Phasenreinheit der Trägermatrix mit definierter Mikroporenstruktur gewährleistet eine vorhersagbare Resorption und damit ein "controlled release" auch der Proteinkomponente A oder einer dafür kodierenden DNA. Eine solche Wirkungsbeziehung zwischen Matrix B und Protein A stellt zweifelsfrei eine synergistische Wirkungsverstärkung der beiden Komponenten A und B dar.

Ein weiterer Gegenstand der vorliegenden Erfindung ist ein Verfahren zur Herstellung der erfindungsgemäßen lmplantatmaterialien, bei dem das Protein A oder die DNA als Lösung in einem physiologisch unbedenklichen, mit Wasser mischbaren Lösungsmittel oder in entsprechenden Lösungsmittelgemischen in die mikroporöse Struktur der biokompatible Matrix B so aufgebracht wird, daß eine homogene Verteilung der Komponente A in und/oder auf der mikroporösen Struktur der Matrix erreicht wird.

Informationen zur Herstellung von Proteinen der TGF-β Superfamilie, deren Expression in geeigneten Wirtszellen und Reinigung sind aus den zahlreichen bereits zitierten Publikationen und Patentschriften zu entnehmen. insbesondere verwiesen sein soll für die Bereitstellung von MP52/GDF-5 oder aktiven Fragmenten davon auf die WO 95/04819 und die DE 19525416.3 sowie auf Hötten et al. (Growth Factors 13 (1996) 65-74).

Erfolgt die Herstellung der Proteine in Bakterien, wobei die Proteine, wie es z.B. bei MP52 der Fall ist, in Form von Einschlußkörpern ("inclusion bodies") vorliegen, werden sie nach an sich bekannten Methoden renaturiert, um das Protein, beispielsweise MP52, in einer aktiven Form zu erhalten. In E.coli exprimierte MP52-ähnliche Proteine können zu einem aktiven Protein zurückgefalten werden vgl.: Krieglstein, K. et al., J. Neuroscience Res. 42 (1995) 724-732. Genaue Verfahren sind auch beschrieben in der japanischen Patentanmeldung Hei-7( ' 95)-93664 sowie in der DE 19525416.3. Weitere eigene Untersuchungen sowie Untersuchungen von Ruppert, R. et al. (Eur. J. Biochem. 237, 295-302 (1996)) haben ergeben, daß z.B. BMP-2 ebenfalls in E.coli exprimiert und zum Dimer gefaltet werden kann.

Die Herstellung der DNA erfolgt nach dem Fachmann bekannten Methoden, wie sie z.B. in Current Protocols in Molecular Biology (Ausubel et al., Greene Publishing Associates and Wiley-Intersience, Wiley & Sons, 1987-1996) oder in Molecular Cloning (Sambrook et al., second edition, Cold Spring Harbor Laboratory Press 1989) beschrieben sind.

Zur Herstellung der Trägermatrix B kann das folgende Verfahren nach DE 38 10 803 C2 verwendet werden: Homogene stöchiometrische Mischungen von CaCO₃ und CaHPO₄ werden entsprechend dem Zustandsdiagramm (Phasendiagramm) des Systems CaO und P₂O₅ (Trömel, Stahl und Eisen 63 (1943) 21 ; Welch, J.

Chem. Soc. (1961) 4442) in kompaktierten Formkörpern in verschiedenen Schritten Sintertemperaturen bis 1350 °C unterworfen, wobei dem Sintersystem Wasser und CO₂ entzogen werden. Zwischen den Sinterprozessen werden die Zwischenstufen der Sintersynthese der Zerkleinerung, Mikronisierung, Rekompaktierung im Fall der Formkörper-Herstellung bzw. Pelletisierung im Fall der Granulat-Herstellung zugeführt. Die Sinterprozesse werden bzgl. Zeit- und Temperaturgang so geführt, daß koexistierende Nachbarphasen des TCP gemäß Phasendiagramm, d.h. insbesondere das Tetra-Calciumphosphat einerseits und das Di-Calciumphosphats andererseits, vermieden werden. Metastabile Phasen des termodynamisch stabilen β-Ca₃(PO₄)₂ oder β-TCP können je nach Verwendungszweck durch Lenkung der Sinterprozesse entweder gezielt vermieden oder gezielt koexistent gemacht oder gar allein vorherrschend dargestellt werden.

Eine homogene Einbringung und Verteilung der Komponente A im Porengefüge der Trägermatrix B setzt einige Verfahrensprinzipien voraus, die eine solche Verteilung ermöglichen, ohne daß die Komponenten selbst durch die Prozesse verändert werden.

So ist ohne weiteres verständlich, daß eine Kombination von A mit B simultan zum keramischen Sinterprozess wegen der hohen Prozesstemperaturen unmöglich ist.

Möglich ist dagegen die Penetration der mikroporösen Keramikgefüge, sowohl Formteile als auch Granulatkörner, mit Lösungen von den erfindungsgemäßen Proteinen A oder der dafür kodierenden DNA in geeigneten Lösungsmitteln, wobei die Kapillarkräfte der offenen Keramikgefüge wirksam werden. Bei der Auswahl der Lösungsmilte) kommen naturgemäß nur solche in Frage, welche die Natur der Komponenten A und B des Biomaterials nicht verändern. So sind zum Beispiel saure Lösungsmittel ungeeignet, welche zwar hervorragende Lösungsmittel für osteopoetische Proteine sind, die Calciumphospate dagegen angreifen und chemisch verändern. Andererseits verhält sich Wasser gegenüber der Keramik neutral, vermag jedoch die Proteinkomponente A oft nur unvollständig zu lösen. Ein Penetrieren mittels Suspensionen dürfte einer homogenen Verteilung in der Trägermatrix wegen der mikroporösen Struktur entgegenstehen.

Ein Penetrationsprozess der Trägermatrix über die Lösungsphase, welche naheliegenderweise durch Verdampfung ausgetrieben wird, führt ebenfalls nicht zur vollkommen homogenen Verteilung von A in B, da sich beim Verdunsten des Lösungsmittels an der Oberfläche der porösen Keramik ein Stofftransport der gelösten Phase von Innen nach Aussen mit der Folge der Anreicherung an der Oberfläche ergibt.

Eine Lösung für diese komplexe Aufgabenstellung einer homogenen Dotierung der Trägermatrix mit der Komponente A kann erfindungsgemäß durch folgende Verfahrensprinzipien bewerkstelligt werden:
- Entfernung des Lösungsmittels nach Abkühlung einer leicht erwärmten, mit den Proteinen A gesättigten Lösung. Hierdurch wird die Löslichkeit der Proteine im Lösungsmittel unterschritten und es kommt zur Abscheidung der Proteine im Gefüge des Trägers. Die Restlösung verarmt dadurch an Protein und der beschriebene Anreicherungseffekt wird entsprechend reduziert. Diese Möglichkeit ist durch den geringen Temperaturspielraum naturgemäß sehr eingeschränkt, macht aber insbesondere dann einen Sinn, wenn man im Hinblick auf einen gewissen "Startereffekt" der Osteopoese einen Gehaltsgradienten des Proteins im Keramikgefüge wünscht.
- Entfernung einer Protein- oder DNA-haltigen Flüssigkeitsmischung, bestehend aus organischem Lösungsmittel und/oder Wasser, durch Sublimation, entsprechend der critical point-Trocknung. Durch den direkten Übergang der erstarrten Lösungsmittelmischung in den gasförmigen Zustand wird ein Transport des Proteins oder der DNA über die Flüssigphase verhindert, wodurch eine gleichmäßige Verteilung des präzipitierten Proteins oder der DNA im Keramikgefüge erreicht wird.
- Homogene Präzipitation von Protein im keramischen Trägergefüge aus einer Protein- enthaltenden organischen Lösung durch Zuführung von z.B. Wasser, wodurch es zu einer raschen Präzipitation des Proteins und somit zur in situ Deposition im Keramikgefüge kommt. Diese Methode ist vielseitig einsetzbar und funktioniert bei Stoffpaarungen, bei denen das Protein ' im organischen Lösungsmittel löslich ist, das reine Lösungsmittel mit Wasser mischbar ist, nicht jedoch die Protein-haltige Lösung. Hierzu können z.B. Acetonitril/Wasser, Propandiol-1,2/Wasser oder Propanol/Wasser u.a. eingesetzt werden.
- Homogene Präzipitation von DNA im keramischen Trägergefüge aus einer DNA-haltigen Salzlösung (zum Beispiel 0,1 M NaCl oder 0,25 M NaAc) durch Zuführung von alkoholischer Lösung, wie beispielsweise absoluter Ethanol, wodurch es zu einer raschen Präzipitation der DNA im Keramikgefüge kommt. Die dotierte Trägermatrix kann bei Bedarf mit 70 % Ethanol gewaschen werden.

Die erfindungsgemäßen Implantatmaterialien können auf ihre Wirksamkeit in gängigen Testsystemen wie z.B. dem bereits erwähnten Tiermodell der Ratte, dem Hund und dem Kaninchen oder aber bei Primaten getestet werden.

Weitere Gegenstände der vorliegenden Erfindung sind daher eine pharmazeutische Zusammensetzung, enthaltend ein Implantatmaterial gegebenenfalls zusammen mit pharmazeutisch wie physiologisch verträglichen Hilfs-, Verdünnungsund/oder Füllstoffen, ebenso wie die Verwendung der erfindungsgemäßen Verbindungen in einer pharmazeutisch wirksamen Konzentration gegebenenfalls zusammen mit pharmazeutisch wie physiologisch verträglichen Hilfs-, Verdünnungs- und/oder Füllstoffen zur Herstellung eines Arzneimittels lokalen Behandlung von Knorpel- und/oder Knochen-Erkrankungen und/oder von Schädigungen des Knorpel- und/oder Knochengewebes verursacht durch Verletzung, Operation, Degeneration oder Überbelastung, bei Wirbeltieren, insbesondere Säugern wie Menschen.

Mit den erfindungsgemäßen Verbindungen können Erkrankungen die mit einem Knochenverlust einhergehen, wie er z.B. bedingt ist durch Alter, Stoffwechselerkrankungen oder entzündliche Prozesse, gezielt behandelt werden.

Schädigungen des Knorpel- oder Knochengewebes sind denkbar nach Verletzungen, beispielsweise auch Sportverletzungen, Unfällen, Überbelastungen des Bewegungsapparates oder können operationsbedingt, beispielsweise durch Bohrlöcher im Knochen nach Entfernung von Schrauben für künstliche Befestigungsapparaturen oder nach Resektion von Tumorgewebe, auftreten. Besonders bevorzugt ist die gezielte lokale Behandlungen von Knochenfrakturen. Möglich sind auch die Verlängerung von Gliedmaßen. Von besonderem Interesse sind auch Anwendungen im Zahn- oder Kieferbereich, wie z.B. die Behandlung von Parodontose, Sinuslift oder Zystenfüllung im Kieferbereich. Anwendungen sind auch in der kosmetischen Chirurgie zu finden, insbesondere in der plastischen Chirurgie im Gesichtsbereich. Die erfindungsgemäßen Verbindungen ermöglichen auch die Fixierung zweier beweglicher Knochenteile wie z.B. die Verbindung zweier Rückenwirbel über eine neu gebildete Knochenbrücke wie es z.B. bei Bandscheibenproblemen von Vorteil sein kann. Umfaßt sind die genannten Behandlungsmethoden ferner im veterinärmedizinischem Bereich.

Die Dosis liegt je nach Art der Proteinkomponente und in Abhängigkeit von der Applikationsart, dem Krankheitsbild und dem Zustand des Patienten im Bereich von 10 µg bis 100 mg. Die Menge der Trägermatrix richtet sich nach der Größe des zu behandelnden Knochen- bzw. Knorpeldefektes.

Bei der Verwendung von größeren gepreßten Trägermatrixes wird die mechanische Befestigung durch z.B. Stahlstangen und -schrauben notwendig.

Mit dem dieser Erfindung zugrundeliegenden synergistischen Effekt durch Kombination von Wirkungsmechanismen zweier Komponenten in einer Verbindung, d.h. knorpel- und/oder knocheninduzierendes Protein und osteoinduktive Trägermatrix, können sehr gute Ergebnisse bei Behandlungen erreicht werden.

Ein Vorteil der erfindungsgemäßen Implantatmaterialien ist die Möglichkeit, Heilungsprozesse, die knorpel- und/oder knocheninduzierende Reaktionen voraussetzen, wesentlich zu verbessern und zu beschleunigen. Dies hat vorteilhafterweise eine deutliche Reduzierung der Leidenszeit bei Patienten, verkürzte Arbeitszeitausfälle und Reduktion der Krankenhausaufenthaltskosten zur Folge. Ein weiterer wirtschaftlicher Aspekt ist gegeben durch die wirkungsvolle Behandlung der Volkskrankheit Parodontose, welche mit dem vorzeitigen Zahnverlust einhergeht. Wirtschaftlich steht somit der durch Parodontose-Behandlung mögliche Zahnerhalt dem kostspieligen vorzeitigen Zahnersatz gegenüber.

Es folgt eine kurze Beschreibung der Figuren:

SEQ ID NO. 1 zeigt die vollständige Aminosäuresequenz des Vorläuferproteins des humanen TGF-β-Proteins MP52. Der Beginn des reifen Proteins liegt vorzugsweise im Bereich der Aminosäuren 361 - 400, besonders bevorzugt bei Aminosäure 381 oder 382. Der reife Proteinanteil enthält die sieben konservierten Cysteine an den Positionen 400, 429, 433, 465, 466, 498 und 500.

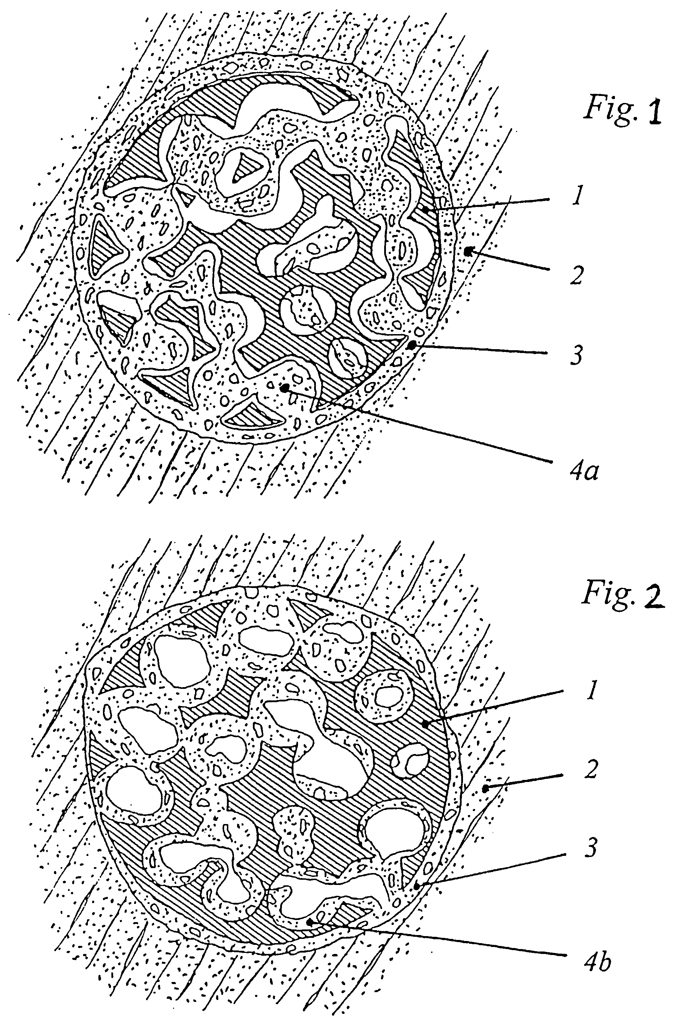

Figur 1 und 2 zeigen die osteoinduktive Wirkung der erfindungsgemäßen Trägermatrix im Vergleich zu einer biokompatiblen, aber nicht bioaktiven Matrix.

In Figur 1 ist die Knochenbildung in den Poren einer bioinerten Matrix, z.B. Al₂O₃ (1 in Fig. 1) schematisch dargestellt. Figur 2 zeigt im Gegensatz dazu die osteoinduktive Wirkung der erfindungsgemäßen Calciumphosphat-Matrix (1 in Fig. 2) in einer sonst identischen Implantationssituation:

In beiden Fällen wurden zylindrische Implantate (1) (Aussen-⌀: 6 mm) mit einer offen durchgängigen Makroporosität (Porendurchmesser ca. 0,5 bis 0,7 mm ⌀) in den kompakten Knochen (Schienbeinknochen eines Hundes) implantiert. Der kompakte Lagerknochen (2) mit deutlich sichtbarer gerichteter "Lamellenstruktur" bildet nach kurzer Implantationszeit um das Implantat neuen Knochen, der den Zwischenraum zwischen Bohrloch im Knochen und den Außenrand des Implantates zu überbrücken (3) sucht. Außerdem bildet sich auch in den Poren des Implantates neuer Knochen (4a in Fig. 1 und 4b in Fig. 2).

Der grundlegende Unterschied zwischen den beiden Implantatmaterialien besteht darin, dass sich der Knochen im osteoinduktiven Implantatmaterial (4b in Fig. 2) unmittelbar und spontan auf den Innenflächen der Poren bildet und die Trägermatrix als Nukleationsort benutzt und von dort aus den gesamten Implantatbereich ausfüllt. Im Gegensatz dazu wächst der Knochen im nicht-bioaktiven Material (Fig. 1) sehr zögernd durch die Porenmitten und vermeidet auch auf Dauer jeden direkten Kontakt mit dem Implantatmaterial. Aus diesen Unterschieden ergibt sich eine sehr intensive und schnelle Verbundbildung beim osteoinduktiven Material (Fig. 2) und eine zögernde, von Osteoklastentätigkeit begleitete unvollständige Verbundbildung beim "bioinerten" Material (Fig. 1).

## Patentansprüche

1. Bioaktives Implantatmaterial für den Knochenersatz mit Knorpelund/oder Knochen-bildender Aktivität bestehend aus zwei Komponenten A und B,
**dadurch gekennzeichnet,**
**dass** A ein auf B aufgebrachtes Protein oder Proteingemisch mit Knorpel- oder/und Knochen-induzierender Aktivität oder für ein oder mehrere derartige Proteine codierende DNA ist und B ein Matrixmaterial aus Calciumphosphat ist, welches in einer für die medizinische Anwendung hergestellten injizierbaren Suspension in geeigneten Flüssigkeiten selbst osteoinduktiv ist.

2. Implantatmaterial nach Anspruch 1, wobei Komponente A ein oder mehrere homo- oder heterodimere Proteine der TGF-β-Superfamilie mit knorpelund/oder knocheninduzierender Aktivität, vorzugsweise der GDF- oder BMP-Familie, oder Fragmente davon umfaßt oder dafür kodierende DNA-Sequenzen.

3. implantatmaterial nach Anspruch 1 oder 2, wobei die Komponente A
(a) den reifen Anteil und gegebenenfalls weitere funktionelle Anteile der in SEQ ID NO. 1 gezeigten Proteinsequenz enthält,
(b) Teile des reifen Anteils von (a) enthält, die im wesentlichen dieselbe Aktivität aufweisen, insbesondere reife Proteine mit veränderten N-Terminus,
(c) Teile entsprechend (a) oder (b) enthält, die aufgrund der Herkunft des Proteins aus anderen Wirbeltieren von SEQ ID NO. 1 abweichen, aber im wesentlichen dieselbe Aktivität aufweisen,
(d) neben Teilen des reifen Proteins gemäß (a), (b) oder (c) noch Teile eines anderen Proteins aus der TGF-β-Superfamilie in Form eines Fusionsproteins enthält,
(e) neben monomeren reifen Proteinen gemäß (a) bis (d) noch ein Monomer eines anderen Proteins aus der TGF-β-Superfamilie unter Ausbildung von Heterodimeren enthält,
(f) neben dimeren reifen Proteinen gemäß (a) bis (e) noch mindestens ein Dimer eines anderen Proteins aus der TGF-β-Superfamilie enthält.

4. Implantatmaterial nach einem der Ansprüche 1 bis 3 , wobei B eine biodegradable oder/und bioaktive Trägermatrix aus Tricalciumphosphat-Keramik bedeutet, welche aus einer kristallographisch phasenreinen α- oder β-Tricalciumphosphat-Keramik miteinerinterkonnektierenden Mikroporosität im Bereich von 20-60 % ihres Volumens besteht und allein bereits knocheninduzierende Eigenschaften besitzt.

5. Implantatmaterial nach Anspruch 4, wobei B eine biodegradable oder/und bioaktive Trägermatrix aus kristallographisch phasenreiner α- oder *β*-Tricalciumphosphat-Keramik bedeutet, deren Primärkorngröße im Bereich von 10-40 µm liegt und die in einer für die medizinische Anwendung hergestellten Suspension in geeigneten Flüssigkeiten wie Wasser, Serum, Plasma und Blut keine Riesenzell- oder Bindegewebeinfiltration in das Implantat verursacht.

6. Implantatmaterial nach Anspruch 4 oder 5,
**dadurch gekennzeichnet,**
**daß** es in Form einer injizierbaren Suspension vorliegt.

7. Implantatmaterial nach Anspruch 4, 5 oder 6, wobei B eine biodegradable und bioaktive Trägermatrix aus kristallographisch phasenreiner α- oder β-Tricalciumphosphat-Keramik bedeutet, welche A in kontrolliert retardierter Weise ("controlled release") in dem Maße freisetzt wie B dem chemischen Abbau im Knochenlager unterliegt.

8. Verfahren zur Herstellung eines bioaktiven Implantatmaterials nach einem der Ansprüche 1 bis 7, bei dem das Protein oder die DNA-Sequenz A als Lösung in einem physiologisch unbedenklichen, mit Wasser mischbaren Lösungsmittel oder in entsprechenden Lösungsmittelgemischen in die mikroporöse Struktur der biokompatible Matrix B so aufgebracht wird, daß eine homogene Verteilung von A in und/oder auf der mikroporösen Struktur der Matrix erreicht wird.

9. Verfahren zur Herstellung einer Verbindung nach Anspruch 8, wobei das Lösungsmittel bzw. Lösungsmittelgemisch durch Sublimation, vorzugsweise durch Gefriertrocknung, entfernt wird.

10. Verfahren zur Herstellung einer Verbindung nach Anspruch 8, wobei das Protein oder die DNA-Sequenz A durch in situ-Präzipitation in der Matrix B aus dem Lösungsmittel durch Zumischen eines präzipitierenden Lösungsmittels, vorzugsweise Wasser oder Ethanol, angereichert wird.

11. Pharmazeutische Zusammensetzung, enthaltend ein Implantatmaterial nach einem der Ansprüche 1 bis 7 gegebenenfalls zusammen mit pharmazeutisch wie physiologisch verträglichen Hilfs-, Verdünnungs- und/oder Füllstoffen.

12. Verwendung eines bioaktiven Implantatmaterials nach einem der Ansprüche 1 bis 7 oder einer pharmazeutischen Zusammensetzung nach Anspruch 11 zur Herstellung eines Arzneimittels zur lokalen Behandlung von Erkrankungen, die den Knorpel und/oder Knochen betreffen, oder/und von Schädigungen des Knorpelund/oder Knochengewebes, die durch Verletzung, Operation, Degeneration oder Überbelastung verursacht wurden.

13. Verwendung eines bioaktiven Implantatmaterials nach einem der Ansprüche 1 bis 7 oder einer pharmazeutischen Zusammensetzung nach Anspruch 11 zur Herstellung eines Arzneimittels zur Behandlung von Knochendefekten wie z.B. Parodontose, Sinuslift, Zystenfüllung im Kieferbereich, Knochenfrakturen, Knochenersatz sowie zum Einsatz in der kosmetischen und plastischen Chirurgie und zur Fixierung beweglicher Knochenteile.

## Claims

1. Bioactive implant material for bone replacement having cartilage-forming and/or bone-forming activity composed of two components A and B,
**wherein**
A is a protein or protein mixture which is applied to B and has a cartilage-inducing or/and bone-inducing activity or a DNA which codes for one or more of such proteins and B is a matrix material composed of calcium phosphate which is itself osteoinductive in an injectable suspension in suitable liquids prepared for the medical application.

2. Implant material as claimed in claim 1, wherein component A comprises one or more homodimeric or heterodimeric proteins of the TGF-β superfamily with a cartilage-inducing and/or bone-inducing activity preferably of the GDF or BMP family or fragments thereof or DNA sequences coding therefor.

3. Implant material as claimed in claim 1 or 2, wherein component A
(a) contains the mature part and optionally additional functional parts of the protein sequence shown in SEQ ID NO. 1,
(b) contains parts of the mature part of (a) which have essentially the same activity, in particular mature proteins with a modified N-terminus,
(c) contains parts corresponding to (a) or (b) which differ from SEQ ID NO: 1 due to the origin of the protein from other vertebrates but have essentially the same activity,
(d) in addition to containing parts of the mature protein according to (a), (b) or (c), also contains parts of another protein from the TGF-β superfamily in the form of a fusion protein,
(e) in addition to containing monomeric mature proteins according to (a) to (d), also contains a monomer of another protein from the TGF-β super-family with formation of heterodimers,
(f) in addition to containing dimeric mature proteins according to (a) to (e), also contains at least one dimer of another protein from the TGF-β super-family.

4. Implant material as claimed in one of the claims 1 to 3, wherein B denotes a biodegradable or/and bioactive carrier matrix composed of a tricalcium phosphate ceramic which is composed of crystallographically phase-pure α-or β-tricalcium phosphate ceramic with an interconnecting microporosity in the range of 20-60 % of its volume and alone already has bone-inducing properties.

5. Implant material as claimed in claim 4, wherein B denotes a biodegradable or/and bioactive carrier matrix composed of a crystallographically phase-pure α- or β-tricalcium phosphate ceramic with a primary particle size in the range of 10-40 µm and in a suitable suspension for a medical application in suitable liquids such as water, serum, plasma and blood, causes no giant cell or connective tissue infiltration into the implant.

6. Implant material as claimed in claim 4 or 5,
**wherein**
it is present in the form of an injectable suspension.

7. Implant material as claimed in claim 4, 5 or 6, wherein B denotes a biodegradable and bioactive carrier matrix composed of a crystallographically phase-pure α- or β-tricalcium phosphate ceramic which releases A in a controlled retarded manner (controlled release) to the extent that B is subjected to chemical degradation in the bone store.

8. Process for the production of a bioactive implant material as claimed in one of the claims 1 to 7 in which the protein or the DNA sequence A is applied in the microporous structure of the biocompatible matrix B as a solution in a physiologically acceptable, water-miscible solvent or in appropriate solvent mixtures in such a way that a homogeneous distribution of A in and/or on the microporous structure of the matrix is achieved.

9. Process for the production of a compound as claimed in claim 8, wherein the solvent or solvent mixture is removed by sublimation preferably by freeze drying.

10. Process for the production of a compound as claimed in claim 8, wherein the protein or the DNA sequence A is concentrated by in situ precipitation in the matrix B from the solvent by admixing a precipitating solvent which is preferably water or ethanol.

11. Pharmaceutical composition containing an implant material as claimed in one of the claims 1 to 7 optionally together with pharmaceutically as well as physiologically acceptable auxiliary substances, diluents and/or fillers.

12. Use of a bioactive implant material as claimed in one of the claims 1 to 7 or of a pharmaceutical composition as claimed in claim 11 to produce a pharmaceutical preparation for the local treatment of diseases which affect cartilage and/or bones or/and of damage to cartilage and/or bone tissue caused by injury, operation, degeneration or strain.

13. Use of a bioactive implant material as claimed in one of the claims 1 to 7 or of a pharmaceutical composition as claimed in claim 11 to produce a pharmaceutical preparation for the treatment of bone defects such as periodontosis, sinus lift, cyst filling in the jaw region, bone fractures, bone replacement as well as for applications in cosmetic and plastic surgery and for immobilizing movable bone parts.

## Revendications

1. Matériau de greffe bioactif pour le remplacement d'os à activité chondroformatrice et/ou ostéoformatrice, constitué de deux composants A et B,
**caractérisé en ce que**,
A est une protéine ou un mélange de protéines, placé(e) sur B, à activité chondroinductrice et/ou ostéoinductrice ou de l'ADN codant pour une ou plusieurs telle(s) protéine(s) et B est un matériel matriciel en phosphate de calcium qui est lui-même ostéoinductif dans une suspension injectable fabriquée pour une utilisation médicale dans des fluides appropriés.

2. Matériau de greffe selon revendication 1, tel que le composant A comprend une ou plusieurs protéines homodimères ou hétérodimères de la superfamille des TGF-β à activité chondroinductrice et/ou ostéoinductrice, de préférence de la famille des GDF ou des BMP, ou des fragments de celles-ci ou des séquences d'ADN codant pour cela.

3. Matériau de greffe selon la revendication 1 ou 2, tel que le composant A
(a) contient la fraction mature et, éventuellement, d'autres fractions fonctionnelles de la séquence protéique représentée dans la SEQ. ID. NO. 1,
(b) contient des parties de la fraction mature de (a) qui présentent essentiellement la même activité, en particulier des protéines matures à N-terminus modifié,
(c) contient des parties correspondant à (a) ou à (b) qui, du fait de la provenance de la protéine d'autres vertébrés, différent de la SEQ ID NO. 1, mais présentent essentiellement la même activité,
(d) contient outre des parties des protéines matures selon (a), (b) ou (c), des parties d'une autre protéine de la superfamille des TGF-β sous la forme d'une protéine de fusion,
(e) contient, outre des protéines monomères matures selon (a) à (d), un monomère d'une autre protéine de la superfamille des TGF-β par formation d'hétérodimères,
(f) contient, outre des protéines dimères matures selon (a) à (e), au moins un dimère d'une autre protéine de la superfamille des TGF-β.

4. Matériau de greffe selon l'une des revendications 1 à 3, tel que B représente une matrice support biodégradable ou/et bioactive en céramique de phosphate tricalcique constituée d'une céramique de phase cristallographiquement pure α- ou β-phosphate tricalcique d'une microporosité interconnectante comprise entre 20% et 60% de son volume et qui possède déjà à elle-seule des propriétés ostéoinductrices.

5. Matériau de greffe selon la revendication 4, tel que B représente une matrice support biodégradable ou/et bioactive en céramique de phase cristallographiquement pure α- ou β-phosphate tricalcique dont la granulométrie primaire est comprise entre 10 et 40 µm et qui ne provoque pas, dans une suspension fabriquée à des fins médicales dans des fluides appropriés tels que eau, sérum, plasma et sang, d'infiltrations de cellules géantes et de tissu conjonctif dans la greffe.

6. Matériau de greffe selon la revendication 4 ou 5,
**caractérisé en ce que**
il se présente sous forme de suspension injectable.

7. Matériau de greffe selon les revendications 4, 5 ou 6, tel que B représente une matrice support biodégradable et bioactive en céramique de phase cristallographiquement pure α- ou β-phosphate tricalcique qui libère A de manière retardée et contrôlée ("controlled release") au fur et à mesure que B est soumis à la décomposition chimique dans le lit osseux.

8. Procédé de fabrication d'un matériau de greffe bioactif selon l'une des revendications 1 à 7, dans lequel la protéine ou la séquence d'ADN A est appliquée, en solution dans un solvant physiologiquement inoffensif, miscible à l'eau ou dans des mélanges de solvants correspondants dans la structure microporeuse de la matrice biocompatible B de manière à obtenir une répartition homogène de A dans et/ou sur la structure microporeuse de la matrice.

9. Procédé de fabrication d'une composition selon la revendication 8, tel que le solvant ou le mélange de solvants est éliminé par sublimation, de préférence par lyophilisation.

10. Procédé de fabrication d'une composition selon la revendication 8, tel que la protéine ou la séquence d'ADN A est enrichie par précipitation in situ dans la matrice B à partir du solvant par addition d'un solvant précipitant, de préférence de l'eau ou de l'éthanol.

11. Composition pharmaceutique contenant un matériau de greffe selon l'une des revendications 1 à 7, éventuellement conjointement à des excipients, diluants et/ou charges tolérables pharmaceutiquement et physiologiquement.

12. Utilisation d'un matériau de greffe bioactif selon l'une des revendications 1 à 7 ou d'une composition pharmaceutique selon la revendication 11 pour la fabrication d'un médicament destiné au traitement local de maladies qui concernent le cartilage et/ou l'os, ou/et de détériorations du tissu chondral et/ou osseux qui ont été provoquées par une blessure, une opération, une dégénération ou une sollicitation excessive.

13. Utilisation d'un matériau de greffe bioactif selon l'une des revendications 1 à 7 ou d'une composition pharmaceutique selon la revendication 11 pour la fabrication d'un médicament destiné au traitement d'anomalies osseuses comme, par exemple, la parodontose, l'élévation de sinus, le comblement de kystes dans la région maxillaire, les fractures osseuses, le remplacement d'os ainsi que la mise en oeuvre en chirurgie cosmétique et plastique et la fixation de parties d'os mobiles.
